Europäisches Patentamt

European Patent Office

Office européen des brevets

(11) Publication number: **0 067 138**
**B1**

(12) **EUROPEAN PATENT SPECIFICATION**

(45) Date of publication of patent specification: **02.10.85**

(21) Application number: **82850115.5**

(22) Date of filing: **18.05.82**

(51) Int. Cl.⁴: **C 08 L 23/08, C 08 J 5/18**

(54) Use of an ethylene polymer composition for the production of film.

(30) Priority: **02.06.81 SE 8103442**

(43) Date of publication of application:
**15.12.82 Bulletin 82/50**

(45) Publication of the grant of the patent:
**02.10.85 Bulletin 85/40**

(84) Designated Contracting States:
**AT BE CH DE FR GB IT LI NL**

(56) References cited:
**DE-A-2 362 049**
**FR-A-2 323 751**

(73) Proprietor: **UNIFOS KEMI AB**
**Box 44**
**S-444 01 Stenungsund (SE)**

(72) Inventor: **Friman, Bo Yngve**
**Varlöksvägen 13**
**S-444 00 Stenungsund (SE)**

(74) Representative: **Schöld, Zaid**
**c/o KemaNobel AB Patents Box 11065**
**S-100 61 Stockholm (SE)**

Courier Press, Leamington Spa, England.

**Description**

The present invention relates to the use of compositions based on ethylene polymers for the production of self-adhesive stretch film. More particularly the invention relates to the use of compositions based on an essentially linear ethylene polymer, having a fairly low density, and polyisobutylene for this purpose.

Self-adhesive films for packaging are well-known and used for wrapping foodstuffs in shops and households, for wrapping goods on pallets and for other purposes. Different materials have been used for the production of such films. Among the polyolefins, conventional polyethylene of low density (PELD) and certain copolymers of ethylene, especially copolymers with vinyl acetate, have predominantly been used. By conventional low density polyethylene is understood polyethylene having a complex, branched molecular structure and a broad molecular weight distribution. Different additives have been used to obtain the desired degree of adhesiveness of the films and polyisobutylene is a well-known additive for this purpose. Self-adhesive films produced from copolymers of ethylene and vinyl acetate and polyisobutylene are for example described in the British patent specification 1,401,880. Hitherto known wrapping films of this kind have a comparatively limited stretchability and this leads to an increased risk of rupture. The films also show a rather large tendency to break due to punctures.

According to the present invention it has been found that a composition based on an essentially linear ethylene polymer, having a fairly low density and a fairly narrow molecular weight distribution, and polyisobutylene is very suitable for the production of self-adhesive, stretchable wrapping films. Films made from these compositions have a very good adhesiveness, without the handleability of the films being impaired. The films further show a very good stretchability and are very flexible, even in a stretched condition, and they have a very low tendency to break due to punctures.

The present invention thus relates to the use of an ethylene polymer for the production of self-adhesive stretch film, the composition essentially comprising an ethylene polymer and at least 0.5 per cent by weight of polyisobutylene, based on the composition. The ethylene polymer in the composition is essentially linear and is a copolymer of ethylene and 1 to 20 per cent by weight, based on the ethylene polymer, of one or several alpha-olefins having 3 to 14 carbon atoms. The ethylene polymer should have a density of not more than 935 kg/m$^3$, a molecular weight distribution, expressed as $M_w/M_n$, of not more than 18 and a molecular weight, $M_w$, not below 25 000.

As has been mentioned, polyisobutylene is a well-known additive for conferring adhesive properties to wrapping films. Polyisobutylene of varying molecular weights have been used for this purpose. In the present compositions polyisobutylene having molecular weights up to about 10 000 can be used. It is, however, preferred to use polyisobutylene having lower molecular weights and the molecular weight should preferably not exceed 3000, as this facilitates the incorporation in the ethylene polymer, and as lower amounts are required for obtaining the same degree of adhesiveness. The molecular weight is suitably within the range 300 to 2000 and preferably within the range 800 to 1800. At least 0.5 per cent by weight of polyisobutylene should be present in the compositions. The upper limit is mainly set in order not to give a too great adhesiveness so that the produced film will be difficult to handle, or impossible to handle at all, and the amount should not exceed 10 per cent by weight. The polyisobutylene is suitably used in an amount of 2 to 10 per cent by weight and preferably in an amount of 3 to 6 per cent by weight, based on the composition.

The ethylene polymers which form the base material in the present compositions are essentially linear, have a low density and a comparatively narrow molecular weight distribution. In this respect they are radically different from conventional, by radical-initiated high pressure polymerization produced polyethylene of corresponding density, which has a branched, complex molecular structure and a broad molecular weight distribution. Ethylene polymers to be used in the present compositions can be prepared by co-ordination polymerization in the presence of metal-organic catalysts, e.g. by low pressure processes in slurry, solution or gas-phase, whereby the polymerization conditions and the catalysts are selected to give an essentially linear polymer. The catalysts can for example be based on chromium or titanium. By linear polymer should be understood that the branching that occurs in the polymer is dependent essentially only on the type and amount of comonomer.

Preferably, ethylene polymers produced by a gas-phase process are used in the present compositions, and particularly such polymers produced by the recently commercialized, so-called Unipol®-process. An example of this process is described in the Swedish patent 7603163-2. The process can be carried out in such a manner, that the polymerization of the monomers occurs in a gas-phase in a fluidized bed of already formed granules of the polymer at a pressure of 5 to 25 kg/cm$^2$ and a temperature of 75 to 125°C. A catalyst based on titanium, magnesium and aluminium as active components, e.g. according to the European patent applications 0 004 645, 0 004 646 and 0 004 647, is injected continuously in the bed in an amount of 100 to 500 ppm, based on the amount of added monomer. The polymerization reaction is carried out in the presence of hydrogen gas.

The ethylene polymers in the present compositions should be essentially linear, and the content of aliphatic branches, having a chain length of more than 12 carbon atoms, should be negligible. The greater part of the branches should in themselves be straight and not branched. The amount of short aliphatic branches in the ethylene polymer should be between 1 and 60 per 1000 carbon atoms in the chain, and suitably between 5 and 30 per 1000 carbon atoms. The length of the branches should be between 1 and 12 carbon atoms, suitably between 1 and 8, and preferably between 1 and 4 carbon atoms. Ethylene polymers of this structure can be prepared by copolymerization of ethylene, and up to 20 per cent by weight, based on the ethylene polymer, of alpha-olefins, having 3 to 14 carbon atoms. One or several such comonomers can be employed. The density of the polymer material should not exceed 935 kg/m³, and is suitably within the range 910 to 935 kg/m³. The amount of comonomer for a given density is adapted with respect to length of the comonomer, or comonomers, and greater amounts are required, when shorter comonomers are employed. The ethylene polymer suitably contains from 1 to 20 per cent by weight comonomer, and preferably from 3 to 10 per cent by weight, and the comonomers are alpha-olefins, having 3 to 14 carbon atoms. The comonomer, or comonomers, are suitably $C_3$ to $C_{10}$ alpha-olefins, and preferably $C_3$ to $C_6$ alpha-olefins. As examples of suitable comonomers can be mentioned propylene, 1-butene, 1-pentene, 1-hexene, 4-methyl-pentene, 1-octene. When the ethylene polymer is a copolymer, containing two or more monomers, besides ethylene, it is suitably a terpolymer of ethylene, propylene, or butene, and one higher olefin.

The molecular weight distribution of the ethylene polymer should not be too broad, if the desired properties of the polymer are to be obtained. The molecular weight distribution can be established by gel chromatography, and is here given as the ratio $M_w/M_n$. The ratio should not exceed 18, and it should be within the range 1 to 18. It is suitably within the range 1 to 10, and preferably within the range 1.5 to 6.

The average molecular weight, $M_w$, should be roughly between 25000 and 500000, and is preferably between 50000 and 250000. Another measure of the molecular weight, used in ethylene polymer technology, is the melt index ($MI_2$, viscosity at 190°C, given as grams of material flowing from a standardized test equipment per 10 minutes). For ethylene polymers in the present compositions the melt index, $MI_2$, according to ISO R 292, should be between 0.05 and 3, preferably between 0.2 and 2.

Other additives such as for example antioxidants can of course also be incorporated in the present compositions. For certain purposes it can be advantageous to blend the present compositions with conventional polyethylene based material for adhesive film in order to deliberately lower the mechanical strength and make the produced film more easy to tear while maintaining the satisfactory adhesiveness. In blends of this kind the conventional adhesive-film material can be incorporated in a weight ratio, with respect to the present composition, of up to 1:1.

The compositions are prepared by mixing the ethylene polymer and the polyisobutylene, and optional other additives, using known mixing technique, for example tumbling, mixing in a ball mill or a Banbury-mixer etc.

Films are produced from the compositions in a known manner by means of a tubular blowing or cold-roll casting. The thickness of the films should be at least 5 µm and should not exceed 50 µm to be suitable as wrapping film. The thickness is suitably within the range 10 to 30 µm and preferably within the range 15 to 25 µm. The produced film can, if desired, be further treated in a known manner. It can e.g. be embossed or corona treated.

Films produced from the compositions are self-adhesive and have excellent stretchability. They are further very flexible when used for products of varying shapes and they have a great capability of withstanding punctures from for example sharp edges and corners on the wrapped products. The films have these satisfactory properties even at comparatively thin thicknesses and they are thus very suitable as wrapping material in many different connections, such as in industrial wrapping of pallets and in wrapping foodstuffs and other products in shops. In contrast to conventional polyethylene-based adhesive films, for which the adhesiveness decreases with increased film thickness and which often lose their property of being adhesive at thicknesses of about 25 µm, the adhesiveness of films produced from the present compositions increases with increased thickness of the film. An advantage with respect to production and handling is that films produced according to the invention are only slightly adhesive directly after the film production and the adhesiveness then develops drastically during the following 24 hours as the adhesive agent migrates to the film surface. The just produced films are thus easy to handle, cut and roll etc.

The invention is further described in the following example, which however, is not intended to limit the same. Parts and per cent relate to parts by weight and per cent by weight, respectively, unless otherwise stated.

Example

A base polymer was prepared from ethylene and about 7 per cent by weight of butene according to the Unipol®-process. The polymer material had the following properties:

| | |
|---|---|
| $MI_2$ (according to ISO R 292) | 1 |
| $M_w/M_n$ | 3.5 |
| Density (according to ASTM-D 2839—69) kg/m³ | 919 |

95 Parts of the base polymer were mixed with 5 parts of polyisobutene, molecular weight 900, in a Banbury-mixer at about 200°C. A 20 μm thick film was produced from the composition by blowing at 235°C, blow ratio 2:1. The extruder that was used had a screw diameter of 70 mm and a 200 mm die.

For comparison a 20 μm thick film was produced in the same manner from a composition based on polyethylene from a high-pressure process. The polyethylene had a density of 922 and a melt index of about 1 and the composition contained 5 per cent by weight of polyisobutylene.

The adhesiveness and the mechanical properties of the produced films were investigated. The adhesiveness was tested in the following manner: pieces of the films were fixed onto blocks and two blocks were placed against each other so that the machine directions of the films coincided. A load weighing 2.10 kg was placed on the blocks for 60 seconds. Thereafter the force required to separate the films was measured by connecting a friction meter via string and pulley and taking the scale reading on a voltameter. The tensile strength was measured according to ISO R 1184 in the length and transverse direction (MD and TD respectively). The puncture resistance was measured according to a modified version of ASTM D 1709. A tensile compressive tester was used and the test probe had a crescent shaped end with a diameter of 12 mm and was 6 mm long. It was pressed against the test specimen at a rate of 500 mm/min. Puncture resistance is defined as the force required to rupture a test specimen and the energy absorbed by the film during rupture. The results are shown below:

| | Adhesiveness scale units | Tensile strength MPa MD/TD | Puncture resistance Joule/mm |
|---|---|---|---|
| Film 1, according to the invention | 55 | 38/31 | 60 |
| Film 2, comparison | 50 | 20/17 | 20 |

## Claims

1. Use of an ethylene polymer based composition, which composition substantially comprises an essentially linear ethylene polymer, which is a copolymer of ethylene and 1 to 20 per cent by weight of one or several alpha-olefins having 3 to 14 carbon atoms, which polymer has a density not higher than 935 kg/m$^3$, a molecular weight distribution not higher than 18, a molecular weight, $M_w$, not below 25000, said polymer containing 1 to 60 short aliphatic branches per 1000 carbon atoms in the chain, and 0.5 to 10 percent by weight of polyisobutylene, having a molecular weight of up to 10000, based on the composition, for the production of self-adhesive stretch film.

2. Use according to claim 1, whereby the polyisobutylene has a molecular weight below 3000.

3. Use according to claim 1 or 2, whereby the polyisobutylene is present in the composition in an amount of 2 to 10 per cent by weight.

4. Use according to any of the preceding claims, whereby the molecular weight, $M_w$, of the ethylene polymer is between 25000 and 500000.

5. Use according to claim 1 or 4, whereby the ethylene polymer has a molecular weight distribution within the range 1 to 10.

## Patentansprüche

1. Verwendung einer Zusammensetzung auf Basis eines Ethylenpolymeren, wobei die Zusammensetzung im wesentlichen ein im wesentlichen lineares Ethylenpolymeres umfaßt, das ein Copolymeres von Ethylen und 1 bis 20 Gew.-% eines oder mehrerer Alpha-Olefine mit 3 bis 14 Kohlenstoffatomen ist, wobei das Polymere eine Dichte nicht höher als 935 kg/m$^3$, eine Molekulargewichtsverteilung nicht höher als 18, ein Molekulargewicht $M_w$ nicht unter 25000 hat, wobei dieses Polymere 1 bis 60 kurze aliphatische Verzweigungen pro 1000 Kohlenstoffatome in der Kette enthält, und 0,5 bis 10 Gew.-% Polyisobutylen mit einem Molekulargewicht bis zu 10000 bezogen auf die Zusammensetzung, zur Erzeugung von selbsthaftendem Stretchfilm.

2. Verwendung gemäß Anspruch 1, wobei das Polyisobutylen ein Molekulargewicht unterhalb 3000 hat.

3. Verwendung gemäß Anspruch 1 oder 2, wobei das Polyisobutylen in der Zusammensetzung in einer Menge von 2 bis 10 Gew.-% vorhanden ist.

4. Verwendung gemäß einem der vorhergehenden Ansprüche, wobei das Molekulargewicht $M_w$ des Ethylenpolymeren zwischen 25000 und 500000 liegt.

5. Verwendung gemäß Anspruch 1 oder 4, wobei das Ethylenpolymere eine Molekulargewichtsverteilung in dem Bereich von 1 bis 10 hat.

**Revendications**

1. Utilisation d'une composition à base d'un polymère d'éthylène, composition comprenant principalement un polymère d'éthylène essentiellement linéaire qui est un copolymère d'éthylène et de 1 à 20% en poids d'une ou de plusieurs alpha-oléfines ayant de 3 à 14 atomes de carbone, ce polymère ayant une masse volumique qui n'est pas supérieure à 935 kg/m$^3$, une répartition du poids moléculaire qui n'est pas supérieure à 18, un poids moléculaire, $M_w$, qui n'est pas inférieur à 25000, ce polymère contenant de 1 à 60 ramifications courtes pour 1000 atomes de carbone dans la chaîne, et entre 0,5 et 10% en poids de polyisobutylène ayant un poids moléculaire pouvant atteindre 10000, par rapport à la composition, pour la fabrication de films autocollants étirables.

2. Utilisation selon la revendication 1 dans laquelle le polyisobutylène a un poids moléculaire inférieur à 3000.

3. Utilisation selon la revendication 1 ou 2, dans laquelle le polyisobutylène est présent dans la composition en une proportion comprise entre 2 et 10% en poids.

4. Utilisation selon l'une quelconque des revendications précédentes, dans laquelle le poids moléculaire, $M_w$, du polymère d'éthylène est compris entre 25000 et 500000.

5. Utilisation selon la revendication 1 ou 4, dans laquelle le polymère d'éthylène présente une répartition des poids moléculaires comprise entre 1 et 10.